# EUROPÄISCHE PATENTSCHRIFT

(11) **EP 0 565 989 B1**
(45) Veröffentlichungstag und Bekanntmachung des Hinweises auf die Patenterteilung: **25.06.1997**
(21) Anmeldenummer: 93105634.5
(22) Anmeldetag: 06.04.1993
(51) Int. Cl.: C08L 97/00, A23K 1/16, A23L 1/302

(54) **Präparate von fettlöslichen Substanzen**
Preparations of fat-soluble substances
Préparations de substances liposolubles

(30) Priorität: 14.04.1992 CH 1238/92; 22.01.1993 CH 186/93
(43) Veröffentlichungstag der Anmeldung: 20.10.1993
(73) Patentinhaber: F. HOFFMANN-LA ROCHE AG, 4002 Basel (CH)
(72) Erfinder: Leuenberger, Bruno, CH-4052 Basel (CH)
(74) Vertreter: Cottong, Norbert A.

(56) Entgegenhaltungen:
- WO-A-91/11110
- DE-A- 1 517 044
- DE-A- 3 837 957
- US-A- 4 228 159
- US-A- 4 666 522
- DATABASE WPI Week 8012, Derwent Publications Ltd., London, GB; AN 80-21757C & SU-A-674 739
- JOURNAL OF FOOD SCIENCE. Bd. 47, 1982, CHICAGO US Seiten 1745 - 1748 G. L. CATIGNANI ET AL 'Antioxidant properties of lignin'

## Beschreibung

Die vorliegende Erfindung betrifft neue, stabile, kaltwasser-dispergierbare, pulverförmige Präparate von fettlöslichen Substanzen, sowie ein Verfahren zu deren Herstellung.

Auf dem Gebiet der menschlichen und tierischen Ernährung spielen kaltwasser-dispergierbare Präparate von fettlöslichen Substanzen, z.B. die fettlöslichen Vitamine, Carotinoide, polyungesättigten Fettsäuren und dergleichen, eine wichtige Rolle. In der Regel sind solche Präparate in Folge ihrer Wasserunlöslichkeit oder auch ihrer mehr oder weniger ausgeprägten Stabilität und Handhabbarkeit in Form von Emulsionen oder Trockenpulvern im Handel. Gemeinsam bei derartigen Präparaten sind die Wirkstoffe, d.h. die fettlöslichen Substanzen, in der Regel mittels einer Matrix-Komponente (Schutzkolloid), z.B. Gummi arabicum oder Gelatine, umhüllt. Diese Matrix-Komponente ist u.a. für den Schutz des Wirkstoffes bzw. für dessen Stabilisierung, für eine optimale Resorption und für die allfällig nötige Wasserdispergierbarkeit des Endpräparates verantwortlich. Als Matrix-Komponente (Schutzkolloid) wird häufig Gelatine verwendet, welche von Warmblütern stammt und demgemäss auch gewisse Nachteile aufweist. Als Beispiele seien hier nur genannt die Tatsache, dass etwa Präparate auf der Basis derartiger Gelatine aus religiösen Gründen nicht weltweit Verwendung finden können, dass diese Gelatine, ohne aufwendige Herstellungsverfahren, und demgemäss auch die damit hergestellten, pulverförmigen Präparate, nicht immer die gewünschte Kaltwasser-Dispergierbarkeit aufweisen usw.

Erfindungsgemäss hat sich nun herausgestellt, dass all diese Nachteile eliminiert werden können, wenn an Stelle von Gelatine von Warmblütern ein wasserlösliches oder wasser-dispergierbares Ligninderivat verwendet wird.

Die erfindungsgemässen, stabilen, kaltwasser-dispergierbaren pulverförmigen Präparate von fettlöslichen Substanzen sind demnach dadurch gekennzeichnet, dass sie als Matrix-Komponente ein wasserlösliches oder wasser-dispergierbares Ligninderivat enthalten.

Die PCT Patentpublikation WO 91/11110 beschreibt eine Methode zum Ueberziehen von Tierfutter, insbesondere in Form von Pellets (Kügelchen), mit einer Zusammensetzung eines Fetts oder Oels und eines Ligninsulfonats. Die Zusammensetzung, die auch noch Additive, wie beispielsweise nicht näher identifizierte Vitamine, enthalten kann, wird auf das Tierfutter gesprüht, wobei das Fett bzw. Oel sowie allfällige Additive in die Kügelchen aufgenommen werden und das Ligninsulfonat eine Schutzhülle um die Kügelchen bildet. Ausserdem ist die Herstellung einer Trockenform der Zusammensetzung durch Erhitzen ("Grillieren") beschrieben.

Die US-Patentchrift 4.666.522 beschreibt stabile Ligninsulfonatenthaltende Emulsionen, die als Bindemittel, insbesondere für Kohle, Baumaterialien (Sand, Asphalt usw.) und Tierfutter, verwendet werden können. Diese Emulsionen werden durch Emulgieren eines hydrophoben Stoffes, z.B. eines Wachses oder Oels, und eines Ligninsulfonat-Konzentrats hergestellt, und können in Trockenpulver, z.B. durch Sprühtrocknung, übergeführt werden. Die Trockenpulver ihrerseits können mit Wasser versetzt werden, um die einsetzbaren stabilen Emulsionen wiederherzustellen. Unter den in Frage kommenden hydrophoben Stoffen und sonstigen Bestandteilen werden Vitamine, Carotinoide und polyungesättigte Fettsäuren nicht erwähnt. Die Funktion des Ligninsulfonats wird als Emulgator und Bindemittel angegeben.

Der Ausdruck "kaltwasser-dispergierbare, pulverförmige Präparate" bedeutet im Rahmen der vorliegenden Erfindung feste Anwendungsformen. Die festen Anwendungsformen liegen in Pulverform vor. Der Ausdruck "fettlösliche Substanzen" umfasst im Rahmen der vorliegenden Erfindung die fettlöslichen Vitamine A, D, E und K, Carotinoide, wie beispielsweise β-Carotin, Astaxanthin, Apocarotenal, Canthaxanthin, Apoester, Citranaxanthin und Zeaxanthin, sowie auch mehrfach ungesättigte (polyungesättigte) Fettsäuren. Dies sind fettlösliche Substanzen, welche in der menschlichen oder tierischen Ernährung eine Rolle spielen. Diese Substanzen sind in Folge ihrer Wasserunlöslichkeit oder auch ihrer mehr oder weniger ausgeprägten Stabilität und Handhabbarkeit in der Regel in Form von Emulsionen oder Trockenpulvern im Handel.

Bei den in den erfindungsgemässen Präparaten vorhandenen Ligninderivaten handelt es sich insbesondere um industriell hergestellte Produkte, welche Ligninsulfonate mit den unterschiedlichsten Kationen enthalten. Von besonderem Interesse sind allerdings Natrium-, Calcium und Ammonium-Ligninsulfonat. Ein erfindungsgemässes Präparat kann als Ligninderivat ein einziges oder ein Gemisch mehrerer Ligninderivate enthalten. Darüber hinaus kann das im erfindungsgemässen Präparat vorhandene Ligninderivat Teil eines industriell hergestellten Produktes darstellen, welches neben Ligninderivaten auch noch weitere Komponenten enthält.

Bekanntlich ist das Biopolymer Lignin einer der Hauptbestandteile von Holz und tritt zusammen mit Cellulose in Pflanzen, besonders in Holz, auf. Je nach Art enthält Holz ca. 16 bis 37% Lignin. Chemisch betrachtet handelt es sich bei Lignin um irreguläre Polymere von methoxylierten Phenylpropan-Monomeren (p-Coumarylalkohol, Coniferylalkohol, Sinapylalkohol usw.) mit einem Molekulargewicht von schätzungsweise mindestens 20 kD. Bei der Herstellung von Cellulose wird in einem ersten Schritt das Holz aufgeschlossen, was in den meisten Fällen durch Behandlung mit Sulfit-Laugen bei 125-180°C erfolgt. Dadurch wird die Cellulose freigesetzt und das Lignin in ein wasserlösliches Derivat, Ligninsulfonat (auch als Sulfit-Lignin bekannt) umgewandelt. In kleinerem Ausmass erfolgt der Holzaufschluss auch durch Behandlung des Holzes mit Natriumhydroxid und Dinatriumtetrasulfid (der "Kraft-Prozess''). Das bei diesem Prozess erhaltene Lignin wird als Kraft-Lignin oder Sulfat-Lignin bezeichnet und ist bei neutralem pH nicht wasserlöslich. Neuere Verfahren zur Cellulose-Gewinnung verwenden zum Holzaufschluss organische Lösungsmittel, z.B. Alkohol, auch mit Wasser gemischt, und das dadurch produzierte Lignin wird als Organosolv-Lignin bezeichnet. Diese Form von Lignin ist ebenfalls nicht wasserlöslich. Es sind zur Zeit vor allem Ligninsulfonate sowie Kraft-Lignine auf dem Markt erhältlich. Vielfach wird nach dem Holzaufschluss die Cellulose abgetrennt und die resultierende Ligninsulfonat-haltige Lösung auf ca. 50% Feststoffgehalt konzentriert und in dieser Form verkauft. Die meisten Hersteller-Firmen bieten ebenfalls pulverförmige Produkte an, welche mittels Sprühtrocknung der Lösungen erhalten worden sind, und diese festen Formen enthalten neben Lignin auch diverse Saccharide in beträchtlichen Mengen. Einige Produzenten stellen aus den primären (rohen) Ligninsulfonaten durch enzymatische Entfernung der Sacchariden und allenfalls durch Reinigung, beispielsweise mittels Ultrazentrifugation, Ligninderivate mit einem relativ hohen Gehalt an Ligninsulfonat(en) her. Die ebenfalls angebotenen Kraft-Lignine können sulfoniert werden, um Wasserlöslichkeit zu erreichen, und die Sulfonierungsprodukte eignen sich als Ligninderivate zur Verwendung in den erfindungsgemässen Präparaten. Kommerzielle Ligninsulfonat-Produkte bestehen typischerweise aus ca. 40-90% Ligninsulfonat und kleineren Mengen von diversen Sacchariden, Asche, Kohlenhydraten, Acetaten, Formaten, Harzen usw., wobei die Zusammensetzung recht stark von der verwendeten Holzqualität abhängt. Solche wasserlösliche Ligninsulfonat-Produkte eignen sich ebenfalls zur Verwendung in den erfindungsgemässen Präparaten. Im allgemeinen können sowohl die Rohprodukte mit einem relativ hohen Gehalt an Sacchariden und weiteren Nebenprodukten als auch die obenerwähnten gereinigten Ligninderivate in den erfindungsgemässen Präparaten verwendet werden, sofern solche Ligninderivate wasserlöslich oder zumindest wasserdispergierbar sind. Beispiele gut geeigneter Ligninderivate sind:
Orzan®S (Natrium-Ligninsulfonat von ITT Rayonier, Stamford, CT, und Seattle, WA, USA);
Orzan®A (Ammonium-Ligninsulfonat von ITT Rayonier); 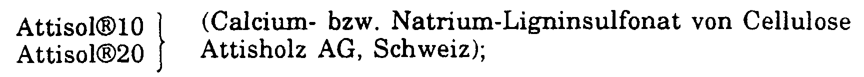
Bei den obigen vier Produkten handelt es sich um primäre Ligninsulfonat-Produkte mit einem relativ hohen Zuckergehalt.
Orzan®LS (Natrium-Ligninsulfonat mit enzymatisch reduziertem Zuckergehalt; ITT Rayonier);
Orzan®CD (Natrium-Ligninsulfonat mit noch reduzierterem Zuckergehalt; ITT Rayonier);
Temsperse®S-001 (Natrium-Ligninsulfonat mit niedrigem Zuckergehalt; von Temfibre Inc., Témiscaming, QC, Kanada);
Tembind®A-001 (Zucker-enthaltendes Ammonium-Ligninsulfonat von Temfibre Inc.);
Reax®910 [(Natrium-) Sulfoniertes Kraft-Lignin von Westvaco Chemicals, Charleston Heights, SC, USA];
Polyfon®O [(Natrium-) Sulfoniertes Kraft-Lignin von Westvaco Chemicals; dieses Produkt muss vor Verwendung auf ca. pH 5,5 gebracht werden, z.B. mittels Salzsäure];
sowie Wafolin® (Zucker-enthaltendes Calcium-Ligninsulfonat von Holmen LignoTech GmbH, Karlsruhe, Deutschland).

Ferner können die erfindungsgemässen Präparate neben dem wasserlöslichen oder wasserdispergierbaren Ligninderivat auch eine zusätzliche Matrix-Komponente, z.B. ein Schutzkolloid, aufweisen. Es eignet sich zu diesem Zwecke insbesondere Gelatine (Gelatine von Warmblütern und/oder Fischgelatine), und zwar deswegen, weil keine Kompatibilitätsprobleme auftreten. Zudem kann das Ligninderivat in Kombination mit einem Trägerstoff, wie beispielsweise Maltodextrin oder einem (zusätzlichen) Zucker, eingesetzt werden.

Das Gewichtsverhältnis der fettlöslichen Substanzen zu den im Endprodukt (im wesentlichen Trockenpulver) letztlich vorhandenen Begleitstoffen (Ligninderivat(e), Wasser, Zucker usw.) beträgt in der Regel etwa 1:100 bis etwa 80:100, wobei die genauen Mengenverhältnisse abhängig sind vom jeweiligen biologischen Bedarf an Wirkstoffen (fettlöslichen Substanzen) und von der Forderung nach gleichmässiger und ausreichend feiner Verteilung der Endpräparate in den zur Konsumation vorgesehenen Anwendungsformen. Sollten auch noch stabilisierende Substanzen in den Präparaten nötig oder erwünscht sein, so können diese in der Regel im Ablauf des Herstellungsverfahrens eingefügt werden.

Die erfindungsgemässen Präparate können im Prinzip dadurch hergestellt werden, dass man eine wässrige Emulsion der fettlöslichen Substanz(en) und des wasserlöslichen oder wasserdispergierbaren Ligninderivats zubereitet und diese Emulsion in ein Trockenpulver überführt. Dieses Herstellungsverfahren stellt einen weiteren Gegenstand der vorliegenden Erfindung dar.

In der Regel werden zunächst alle Bestandteile, also u.a. das Ligninderivat, ausser der fettlöslichen Substanz(en) in Wasser gelöst, was zweckmässigerweise durch kräftiges Rühren beschleunigt wird. Zudem erweist es sich als vorteilhaft, diesen Verfahrensschritt bei erhöhter Temperatur, insbesondere im Temperaturbereich von 20°C bis 90°C, durchzuführen. Auf diese Weise wird die sogenannte Matrix erhalten. Dann wird die fettlösliche Substanz bzw. ein Gemisch mehrerer solchen Substanzen in diese Matrix hineinemulgiert, und zwar ebenfalls vorteilhaft unter kräftigem Rühren oder auch mittels Ultraschall oder ähnlicher Techniken. Der Druck und die Temperatur sind bei diesem Vorgang keine kritischen Parameter, und das Ganze kann ohne weiteres bei Temperaturen von etwa Raumtemperatur bis etwa 70°C und Atmosphärendruck durchgeführt werden. Bei der Herstellung der Emulsion können selbstverständlich normalerweise in derartigen Präparaten üblichen Hilfsstoffe verwendet werden, wie beispielsweise Zucker, z.B. Saccharose; Zuckeralkohole; Stärkederivate, z.B. Maltodextrin; Milchproteine, z.B.Natriumkaseinat; oder auch pflanzliche Proteine, z.B. Sojaprotein, Kartoffelprotein und Weizenprotein. Sollten auch noch stabilisierende Substanzen in den so hergestellten Präparaten nötig oder erwünscht sein, so können diese in der Regel in der Oelphase, d.h. in einer einzufügenden flüssigen fettlöslichen Substanz, gelöst werden. Ferner können bei der Herstellung der Emulsion Emulgatoren, wie beispielsweise Ascorbylpalmitat und Zuckerester, verwendet werden.

Die Ueberführung einer so hergestellten Emulsion, die je nach Bestandteilen im allgemeinen von ca. 40 bis ca. 60 Gewichtsprozent an fettlöslicher(en) Substanz(en) enthält, in Trockenpulver, kann z.B. durch normale Sprühtrocknung, das Doppeldispergier-Verfahren oder auch das Stärke-Catch-Verfahren erfolgen. Bei letzterem Verfahren werden die versprühten Emulsionströpfchen in einem Stärkebett aufgefangen und anschliessend der Trocknung zugeführt. Falls notwendig, kann die zu versprühende Emulsion mit Wasser verdünnt werden. In solchen Fällen kann die Emulsion für praktische Zwecke so wenig wie ca. 1 Gewichtsprozent an fettlöslicher(en) Substanz(en) enthalten.

Die vorliegende Erfindung betrifft ferner die Verwendung eines wasserlöslichen oder wasserdispergierbaren Ligninderivats als Matrix-Komponente zur Herstellung von stabilen, kaltwasser-dispergierbaren, pulverförmigen Präparaten der in Frage kommenden fettlöslichen Substanzen.

Die Herstellung der erfindungsgemässen Präparate ist im allgemeinen einfacher als diejenige herkömmlicher Präparate mit anderen Matrix-Komponenten, z.B. Gelatine. Dies liegt an der guten Wasserlöslichkeit der erfindungsgemäss verwendeten Ligninderivate sowie an ihrer hervorragenden Emulgierfähigkeit. Die gute Wasserlöslichkeit ermöglicht eine signifikante Senkung des Wasseranteils der Emulsion, was zu Einsparungen bei der Produktionskosten und/oder zu einer Erhöhung der Produktionskapazität führt. Die gute Emulgierfähigkeit zusammen mit dem relativ niedrigen Wassergehalt der Emulsion und der dadurch bedingten relativ hohen Viskosität führt zu einer sehr feinen inneren Phase der Emulsion mit einer äusserst engen Partikelgrössenverteilung, was eine sehr gute Stabilität der Emulsion und eine verbesserte Bioverfügbarkeit der darin enthaltenen fettlöslichen Substanzen bewirkt. Ferner weisen die erfindungsgemässen Präparate einen relativ geringen Anteil der jeweiligen fettlöslichen Substanz an der Oberfläche der Partikel auf, was auf die Qualität der Einbettung der fettlöslichen Substanz in der Matrix, d.h. auf das gute Umhüllungsvermögen des verwendeten Ligninderivats, hindeutet.

Im allgemeinen weisen die erfindungsgemässen Präparate eine gute Kaltwasser-Dispergierbarkeit sowie eine gute Fliessbarkeit auf. Ferner hat es sich erwiesen, dass die Präparate relativ niedrige Hygroskopizität besitzen.

Die erfindungsgemässen Präparate können sowohl für die Tierernährung als auch für die menschliche Ernährung Verwendung finden. In gewissen Fällen kann es auch zweckmässig sein, die hergestellten Emulsionen nicht erst in Trockenpulver überzuführen, sondern direkt als solche zu verwenden. Vorzugsweise eignen sich die erfindungsgemässen Präparate für die Formulierung von fettlöslichen Substanzen auf dem Gebiet der Tierernährung.

Die vorliegende Erfindung wird durch die nachfolgenden Beispiele veranschaulicht.

### Beispiel 1

In einem Reaktionsgefäss werden 197,4 g Orzan®S (Natrium-Ligninsulfonat von ITT Rayonier, Stamford, CT und Seattle, WA, USA) in 99 g Wasser bei 50°C unter Verwendung eines mit einer Dissolverscheibe ausgestatteten Rührers gelöst, wobei die Rührgeschwindigkeit 2000 UpM beträgt. Bei einer Rührgeschwindigkeit von 5000 UpM werden zu der resultierenden Lösung 222,6 g auf 70°C erwärmtes dl-α-Tocopherolacetat (Roche) innert 5 Minuten zugegeben. Die resultierende Emulsion wird während 30 Minuten bei 50°C weiter emulgiert (5000 UpM). Diese Emulsion besitzt eine Partikelgrösse der inneren Phase ( Tocopherolacetat) von 193±49nm (Laserdiffraktion: PCS-Spektrometer Coulter N4S, France). Die Emulsion wird mit 692 g Wasser verdünnt und unter Verwendung eines Sprühtrockners (Minor der Firma Niro Atomizer, Dänemark) sprühgetrocknet (Lufttemperatur: Eintritt 220°C, Austritt 72°C). Auf diese Weise werden ca. 300 g Pulver erhalten, dessen Fliessbarkeit durch Untermischen von 1% Kieselsäure Sipernat®50S verbessert wird. Das Produkt besitzt einen Gehalt an Tocopherolacetat von 53,9%. 7,3% des gesamten Tocopherolacetats befinden sich an der Oberfläche der Pulver-Teilchen und sind daher mit Cyclohexan abwaschbar. Das sprühgetrocknete Pulver besitzt gute Fliesseigenschaften und zeigt eine ausgezeichnete Kaltwasser-Dispergierbarkeit.

### Beispiel 2

Analog dem in Beispiel 1 beschriebenen Verfahren wird unter Verwendung von 197,4 g Attisol®10 (Calcium-Ligninsulfonat von Cellulose Attisholz AG, Schweiz), 222,6 g dl-α-Tocopherolacetat und 110 g Wasser eine Emulsion hergestellt. Die Partikelgrösse der inneren Phase beträgt hier 203nm. Die Emulsion wird mit 730 g Wasser verdünnt und sprühgetrocknet. Das pulverförmige Produkt enthält 49,0% Tocopherolacetat, wovon 4,9% mit Cyclohexan abwaschbar sind.

### Beispiel 3

Analog dem in Beispiel 1 beschriebenen Verfahren wird unter Verwendung von 197,4 g Orzan®CD (Natrium-Ligninsulfonat, ITT Rayonier), 222,6 g dl-α-Tocopherolacetat und 160 g Wasser eine Emulsion hergestellt, die eine Partikelgrösse der inneren Phase von 211±50nm aufweist. Nach Verdünnung mit 655 g Wasser wird die Emulsion sprühgetrocknet. Das sprühgetrocknete Pulver besitzt einen Gehalt von 52,0% Tocopherolacetat, wovon 69% mit Cyclohexan abwaschbar sind.

Der abwaschbare Anteil Tocopherolacetat ist bei diesem Produkt erheblich höher als in Beispiel 1 der Fall ist. Dies zeigt, dass unterschiedliche Ligninsulfonat-Qualitäten unterschiedliche Produkte ergeben.

### Beispiel 4

Analog dem in Beispiel 1 beschriebenen Verfahren werden 127,1 g Orzan®LS (Natrium-Ligninsulfonat, ITT Rayonier), 31,2 g einer 45%-igen wässrigen Lösung von Fischgelatine (Norland Products Inc., New Brunswick, NJ, USA), 159 g dl-α-Tocopherolacetat und 100 g Wasser zu einer Emulsion verarbeitet, deren innere Phase eine Partikelgrösse von 467±160nm aufweist. Nach Zugabe von 502 g Wasser wird die Emulsion sprühgetrocknet. Das erhaltene Produkt besitzt einen Gehalt an Tocopherolacetat von 53,6%, wovon 33% mit Cyclohexan abwaschbar sind.

### Beispiel 5

Analog dem in Beispiel 1 beschriebenen Verfahren werden 46,8 g Orzan®S (Natrium-Ligninsulfonat, ITT Rayonier), 141,2 g Maltodextrin MD05 (Roquette, Frankreich), 212 g dl-α-Tocopherolacetat und 94 g Wasser zu einer Emulsion verarbeitet. Die innere Phase dieser Emulsion besitzt eine Partikelgrösse von 337±86nm. Nach Zugabe von 650 g Wasser wird die Emulsion sprühgetrocknet. Das pulverförmige Produkt enthält 53,0% Tocopherolacetat, wovon 17,7% mit Cyclohexan abwaschbar sind.

### Beispiel 6

Analog dem in Beispiel 1 beschriebenen Verfahren wird unter Verwendung von 113,4 g Orzan®S (Natrium-Ligninsulfonat, ITT Rayonier), 306,6 g dl-α-Tocopherolacetat und 57,0 g Wasser eine Emulsion produziert, deren Partikelgrösse der inneren Phase 277±85nm beträgt. Die Sprühtrocknung der Emulsion erfolgt nach Verdünnung mit 783 g Wasser. Das sprühgetrocknete Pulver besitzt einen Gehalt von 74,3% Tocopherolacetat, wovon 52% mit Cyclohexan abwaschbar sind.

Der abwaschbare Anteil Tocopherolacetat ist - bedingt durch den höheren Gehalt an Tocopherolacetat - in diesem Beispiel wesentlich höher als in Beispiel 1 der Fall ist.

### Beispiel 7

In einem Reaktionsgefäss (1 l) werden 87,8 g Orzan®S (Natrium-Ligninsulfonat, ITT Rayonier) in 60,0 g Wasser bei 45°C unter Verwendung eines mit einer Dissolverscheibe ausgestatteten Rührers gelöst, wobei die Rührergeschwindigkeit 2000 UpM beträgt. Während 30 Minuten werden in einem 500 ml-Erlenmeyerkolben 16,5 g β-Carotin (kristallin, Roche) sowie 3,3 g Ethoxyquin in 230 ml Chloroform bei 65°C gelöst. Diese zweite Lösung wird innert 12 Minuten in die warme erste Lösung im 1 l-Reaktionsgefäss unter Rühren bei 5000 UpM einemulgiert. Die resultierende Emulsion wird mit zusätzlichen 50 g Wasser verdünnt und während 30 Minuten nachemulgiert bei 5000 UpM. Die Partikelgrösse der inneren Phase (β-Carotin in Chloroform) dieser Emulsion beträgt 173±42nm. Das Chloroform wird mittels eines Rotationsverdampfers bei 50°C am Wasserstrahlvakuum abdestilliert. Die Partikelgrösse der inneren Phase (β-Carofin) dieser Emulsion beträgt nun 136±51nm.

In einer Laborsprühwanne werden 900 g mittels Kieselsäure fluidisierte und auf ca. 5°C gekühlte Maisstärke vorgelegt. Die Emulsion wird in diese Stärke versprüht, und die erhaltenen mit Stärke umhüllten Partikel werden von der Stärke abgesiebt und bei Raumtemperatur getrocknet. Das pulverförmige Produkt besitzt einen Gehalt an β-Carotin von 10,2% und zeigt gute Kaltwasser-Dispergierbarkeit. 0,1% des β-Carotins dieses Produktes sind mit Methylenchlorid abwaschbar. Die Farbintensität (Absorption) in Wasser dieses Produkts beträgt 1100 (1% Lösung, 1 cm Schichtdicke).

### Beispiel 8

Analog dem in Beispiel 7 beschriebenen Verfahren wird unter Verwendung von 175,6 g Attisol®10 (Calcium-Ligninsulfonat, Cellulose Attisholz), 150 g Wasser, 32,5 g Canthaxanthin (kristallin, Roche), 3,0 g Ethoxyquin sowie 350 ml Chloroform eine Emulsion hergestellt. Nach Zugabe weiterer 59 g Wasser und Entfernung des Chloroform mittels eines Rotationsverdampfers wird die Emulsion/Suspension durch Versprühen in einer Laborsprühwanne in ein Pulver übergeführt. Das Pulver enthält 13,2% Canthaxanthin, wovon 0,2% mit Chloroform abwaschbar ist. Die Teilchengrösse der inneren Phase (Canthaxanthin) beträgt 140±34 nm, und das Produkt weist eine Farbintensität in Wasser von 1272 auf (1% Lösung, 1 cm Schichtdicke).

### Beispiel 9

Analog dem in Beispiel 7 beschriebenen Verfahren wird unter Verwendung von 175,6 g Orzan®S (Natrium-Ligninsulfonat, ITT Rayonier), 180 g Wasser, 32 g Citranaxanthin (kristallin, Roche), 4,9 g Ethoxyquin sowie 510 ml Chloroform eine Emulsion hergestellt. Nach Zugabe von 40 ml Wasser wird das Chloroform am Rotationsverdampfer entfernt. In diese Emulsion wird zusätzlich eine Lösung von 32 g Citranaxanthin und 4,9 g Ethoxyquin in 510 ml Chloroform einemulgiert. Nach erneutem Entfernen des Chloroforms am Rotationsverdampfer wird die Emulsion mittels einer Laborsprühwanne analog Beispiel 7 in ein Pulver übergeführt. Das resultierende Pulver enthält 17,8% Citranaxanthin, wovon 0,3% mit Chloroform abwaschbar ist. Die Teilchengrösse der inneren Phase (Citranaxanthin) beträgt 215±59 nm und das Produkt weist eine Farbintensität in Wasser von 1172 auf (1% Lösung, 1 cm Schichtdicke).

### Beispiel 10

Analog dem in Beispiel 1 beschriebenen Verfahren wird unter Verwendung von 197,4 g Orzan®A (Ammonium-Ligninsulfonat, ITT Rayonier), 222,6 g dl-α-Tocopherolacetat und 99 g Wasser eine Emulsion hergestellt, welche nach Zugabe weiterer 692 g Wasser sprühgetrocknet wird. Das resultierende Pulver enthält 53% dl-α-Tocopherolacetat, wovon 8% mit Cyclohexan abwaschbar sind. Die Teilchengrösse der inneren Phase (dl-α-Tocopherolacetat) der mit Wasser rekonstituierten Emulsion beträgt 198±52 nm.

### Beispiel 11

Analog dem in Beispiel 1 beschriebenen Verfahren wird unter Verwendung einer 45°C warmen Lösung von 200 g Attisol®10 (Calcium-Ligninsulfonat, Cellulose Attisholz) in 90 g Wasser und einer 60°C warmen Lösung, enthaltend 82,5 g Vitamin A Mischung Feed Grade Typ B (Roche) und 3,6 g Ethoxyquin, eine Emulsion hergestellt. Nach Zugabe von 196 g Wasser wird diese Emulsion sprühgetrocknet. Das resultierende Pulver enthält 501 122 IU/g Vitamin A, wovon 0,03% mit Cyclohexan abwaschbar ist. Die innere Phase (Vitamin A) weist eine Teilchengrösse von 126±34 nm auf.

### Beispiel 12

Analog dem in Beispiel 11 beschriebenen Verfahren wird unter Verwendung von 200 g Attisol®10 (Calcium-Ligninsulfonat, Cellulose Attisholz), 112 g Vitamin A Mischung Feed Grade Typ B (Roche) sowie 100 g Wasser eine Emulsion hergestellt. Nach Zugabe von 110 g Wasser wird diese Emulsion mittels einer Laborspührwanne in Maisstärke versprüht, und zwar analog Beispiel 7. Das resultierende Produkt enthält 585 655 IU/g Vitamin A, wovon 0,1% mit Cyclohexan abwaschbar ist. Die Teilchengrösse der inneren Phase (Vitamin A) beträgt 156±42 um.

### Beispiel 13

Analog dem in Beispiel 1 beschriebenen Verfahren wird unter Verwendung einer 45°C warmen Lösung von 200 g Orzan®S (Natrium-Ligninsulfonat, ITT Rayonier) in 90 g Wasser sowie einer 65°C warmen Lösung von 6,18 g Vitamin D₃ (technische Qualität, Roche) und 3,09 g Ethoxyquin in 6,18 g Arachisöl eine Emulsion hergestellt. Nach Zugabe von weiteren 125 g Wasser wird die Emulsion sprühgetrocknet. Das erhaltene Pulver weist einen Vitamin D₃-Gehalt von 488 000 IU/g auf. Die Teilchengrösse der inneren Phase (Vitamin D₃ in Arachisöl) beträgt 96±24 nm.

### Beispiel 14

Analog dem in Beispiel 1 beschriebenen Verfahren wird unter Verwendung von 100 g Reax®910 [(Na-) Sulfoniertes Kraft-Lignin, Westvaco Chemicals, Charleston Heights, SC, USA], 112,5 g dl-α-Tocopherolacetat und 105 g Wasser eine Emulsion hergestellt. Nach Zugabe weiterer 298 g Wasser wird eine Emulsion mit einer Teilchengrösse der inneren Phase von 140±38 nm erhalten. Die erhaltene Emulsion kann als Emulsion belassen werden, oder sie kann analog Beispiel 1 sprühgetrocknet werden.

### Beispiel 15

Analog dem in Beispiel 1 beschriebenen Verfahren wird unter Verwendung von 100 g Temsperse®S-001 (Natrium-Ligninsulfonat, Temfibre Inc., Témiscaming, QC, Kanada), 112,8 g dl-α-Tocopherolacetat und 80 g Wasser eine Emulsion hergestellt. Nach Zugabe weiterer 323 g Wasser resultiert eine wässrige Tocopherolacetat-Emulsion mit einer Teilchengrösse der inneren Phase von 183±55 nm.

## Patentansprüche

1. Stabile, kaltwasser-dispergierbare, pulverförmige Präparate von fettlöslichen Substanzen, dadurch gekennzeichnet, dass sie als Matrix-Komponente ein wasserlösliches oder wasserdispergierbares Ligninderivat und als fettlösliche Substanz(en) eine oder mehrere der Vitamine A, D, E und K, Carotinoide und polyungesättigten Fettsäuren enthalten.

2. Präparat nach Anspruch 1, dadurch gekennzeichnet, dass es als fettlösliche Substanz(en) ein oder mehrere der Carotinoide β-Carotin, Astaxanthin, Apocarotenal, Canthaxanthin, Apoester, Citranaxanthin und Zeaxanthin enthält.

3. Präparat nach Anspruch 1 oder 2, dadurch gekennzeichnet, dass das Ligninderivat Natrium-, Calcium- oder Ammonium-Ligninsulfonat, oder ein Gemisch von zwei oder drei dieser Ligninderivate ist.

4. Präparat nach Anspruch 3, dadurch gekennzeichnet, dass das Ligninderivat Teil eines industriell hergestellten Produkts darstellt, welches neben Ligninsulfonat(en) noch weitere Komponenten enthält.

5. Verfahren zur Herstellung von stabilen, kaltwasser-dispergierbaren, pulverförmigen Präparaten von fettlöslichen Substanzen gemäss Anspruch 1, dadurch gekennzeichnet, dass man eine wässrige Emulsion der fettlöslichen Substanz(en) und des wasserlöslichen oder wasserdispergierbaren Ligninderivats zubereitet und diese Emulsion in ein Trockenpulver überführt.

6. Verfahren nach Anspruch 5, dadurch gekennzeichnet, dass man alle Bestandteile ausser der fettlöslichen Substanz(en) in Wasser löst, um eine Matrix zu erhalten, die fettlösliche Substanz bzw. ein Gemisch mehrerer solchen Substanzen in diese Matrix hineinemulgiert und die so hergestellte Emulsion durch normale Sprühtrocknung, das Doppeldispergier-Verfahren oder das Stärke-Catch-Verfahren in Trockenpulver überführt.

7. Verwendung eines wasserlöslichen oder wasserdispergierbaren Ligninderivats als Matrix-Komponente zur Herstellung von stabilen, kaltwasser-dispergierbaren, pulverförmigen Präparaten von fettlöslichen Substanzen, die als fettlösliche Substanz(en) eine oder mehrere der Vitamine A, D, E und K, Carotinoide und polyungesättigten Fettsäuren enthalten.

## Claims

1. Stable, cold water-dispersible, pulverous preparations of fat-soluble substances, characterized in that they contain a water-soluble or water-dispersible lignin derivative as the matrix component and one or more of vitamins A, D, E and K, carotenoids and polyunsaturated fatty acids as the fat-soluble substance(s).

2. A preparation according to claim 2, characterized in that it contains one or more of the carotenoids β-carotene, astaxanthin, apocarotenal, canthaxanthin, apoester, citranaxanthin and zeaxanthin as the fat-soluble substance(s).

3. A preparation according to claim 1 or 2, characterized in that it contains sodium, calcium or ammonium ligninsulphonate or a mixture of two or three of these lignin derivatives as the lignin derivative.

4. A preparation according to claim 3, characterized in that the lignin derivative is part of an industrially produced product which contains further components in addition to ligninsulphonate(s).

5. A process for the manufacture of stable, cold water-dispersible, pulverous preparations of fat-soluble substances in accordance with claim 1, characterized by producing an aqueous emulsion of the fat-soluble substance(s) and the water-soluble or water-dispersible lignin derivative and converting this emulsion into a dry powder.

6. A process according to claim 5, characterized in that all components except the fat-soluble substance(s) are dissolved in water in order to obtain a matrix, the fat-soluble substance or a mixture of several such substances is emulsified in this matrix and the thus-produced emulsion is converted into a dry powder by normal spray-drying, the double dispersion process or the starch-catch process.

7. The use of a water-soluble or water-dispersible lignin derivative as a matrix component for the manufacture of stable, cold water-dispersible pulverous preparations of fat-soluble substances, which contain as the fat-soluble substance(s) one or more of vitamins A, D, E and K, carotenoids and polyunsaturated fatty acids.

## Revendications

1. Préparations pulvérulentes stables, dispersables dans l'eau froide, de substances liposolubles, caractérisées en ce qu'elles contiennent comme composant matrice un dérivé de lignine soluble dans l'eau ou dispersable dans l'eau, et comme substance(s) liposoluble(s) une ou plusieurs des vitamines A. D, E et K, des caroténoïdes et/ou des acides gras polyinsaturés.

2. Préparation selon la revendication 1, caractérisée en ce qu'elle contient comme substance(s) liposoluble(s) un ou plusieurs des caroténoïdes β-carotène, astaxanthine, apocaroténal, canthaxanthine, apoester, citranaxanthine et zéaxanthine.

3. Préparation selon la revendication 1 ou 2, caractérisée en ce que le dérivé de lignine est le ligninesulfonate de sodium, de calcium ou d'ammonium, ou un mélange de deux ou trois de ces dérivés de lignine.

4. Préparation selon la revendication 3, caractérisée en ce que le dérivé de lignine représente une partie d'un produit industriellement préparé, qui outre le(s) ligninesulfonate(s) contient encore d'autres composants.

5. Procédé de préparation de préparations pulvérulentes stables, dispersables dans l'eau froide, de substances liposolubles selon la revendication 1, caractérisé en ce qu'on prépare une émulsion aqueuse de la (des) substance(s) liposoluble(s) et du dérivé de lignine soluble dans l'eau ou dispersable dans l'eau et en ce qu'on transforme cette émulsion en une poudre sèche.

6. Procédé selon la revendication 5, caractérisé en ce qu'on dissout tous les composants en-dehors de la (des) substance(s) liposoluble(s) dans l'eau, pour obtenir une matrice, en ce qu'on émulsifie la substance liposoluble ou un mélange de plusieurs de ces substances dans cette matrice, et en ce qu'on transforme l'émulsion ainsi préparée en poudre sèche par atomisation normale, par le procédé de double dispersion ou le procédé de fixation d'amidon.

7. Application d'un dérivé de lignine soluble dans l'eau ou dispersable dans l'eau comme composant de matrice pour la préparation de préparations pulvérulentes stables, dispersables dans l'eau froide, de substances liposolubles, qui contiennent comme substance(s) liposoluble(s) une ou plusieurs des vitamines A, D, E et K, des caroténoïdes et des acides gras polyinsaturés.
